# EUROPEAN PATENT APPLICATION

(11) **EP 3 037 124 A1**
(43) Date of publication of application: **29.06.2016**
(21) Application number: 14199769.2
(22) Date of filing: 22.12.2014
(51) Int. Cl.: A61M 37/00

(54) **Drug delivery device with microneedles**

(71) Applicant: Université Libre de Bruxelles, 1050 Bruxelles (BE)
(72) Inventor: Sausse, Marion, 1410 WATERLOO (BE); Deleers, Michel, 1630 LINKEBEEK (BE); Tailler, Serge, 1030 Schaerbeek (BE)
(74) Representative: Pronovem

(57) **Abstract**

Drug delivery assembly comprising: micro needles (151) capable of transdermal fluid delivery, a reservoir (13) containing the drug (14) and fluidly coupled to the micro needles, a plunger (132) coupled to the reservoir and operable to expel the drug from the reservoir, a housing (11) arranged to receive the reservoir, an actuation member (12) moveable relative to the housing (11), means (19) for storing energy, first retention means (17) configured for maintaining the reservoir (13) in a fixed position relative to the housing (11), and second retention means (18) configured for maintaining the plunger (132) in a fixed position relative to the reservoir (13). The first and second retention means are configured to change state in sequence, which releases respectively (A) the reservoir (13) from the housing (11) and (B) the plunger (132) from the reservoir (13). The means (19) for storing energy are operably coupled to the plunger (132) to provide a force on the plunger for moving the micro needles (151) against the skin upon the change of state (A) of the first retention means, as well as for moving the plunger to expel the drug (14) from the reservoir upon the change of state (B) of the second retention means.

## Description

The present invention is related to a drug delivery device for transdermal delivery of a liquid formulation or composition using micro needles.

A drug delivery device of the above kind is known from WO 2012/126784. In relation to Fig. 7 of that document, a device is described which receives a cartridge containing a drug formulation to be administered. The cartridge is in fluid communication with a patch of micro needles at one end and has a movable piston at the opposite end. The piston is actuated by releasing a loaded compression spring, which in turn moves a plunger in abutment against the piston and further moves the piston down the cartridge to empty its contents through the micro needle patch.

The micro needles of the above device must be applied manually on the skin and the impact force of the micro needles with the skin cannot be controlled. This problem is obviated in the drug delivery devices described in WO 2011/014514, US 2012/0109066 and WO 2013/036602. All these devices comprise a first spring member which is coupled to a patch of micro needles, and a second spring member, which is coupled to a piston of a liquid drug containing cartridge. Triggering an activation member of the device will first release energy from the first spring member to create a controlled force for moving the micro needles against the skin at a controlled speed, independent of the triggering action. The energy stored in the second spring member is only released once the micro needles are against the skin, so that the cartridge can be safely emptied. Different systems are described to ensure that the second spring member is activated only after activation of the first spring member.

The vast majority of devices of the above kind will be disposable and hence be used only once. In such disposable devices, cost of manufacture and material usage are key aspects.

It is therefore an object of the invention to provide an assembly or device for drug delivery with micro needles, which is more economical than prior art devices. It is an object to provide an assembly or device for drug delivery which is easier to manufacture, of less complex structure and/or which uses less material.

According to aspects of the invention, there is therefore provided an assembly or device for drug delivery as set out in the appended claims. Assemblies or devices according to aspects of the invention comprise a plurality of micro needles, which are advantageously capable of transdermal fluid delivery and advantageously arranged on a patch, a reservoir containing the drug and arranged to be fluidly coupled to the micro needles or the patch, a plunger or piston coupled to the reservoir and operable to expel the drug from the reservoir, a housing arranged to receive the reservoir and the patch, and means for releasably storing energy. Assemblies or devices also comprise first retention means configured for maintaining the reservoir in an advantageously fixed position relative to the housing, thereby securing the reservoir to the housing, and second retention means configured for maintaining the plunger in an advantageously fixed position relative to the reservoir, thereby securing the plunger to the reservoir. The first retention means are configured to change state. The change of state of the first retention means releases the reservoir from the housing. The second retention means are configured to change state. The change of state of the second retention means releases the plunger from the reservoir. The change of state of the first retention means is advantageously configured to occur prior to the change of state of the second retention means.

According to aspects of the invention, the means for storing energy are operably coupled to the plunger to advantageously continuously provide a force on the plunger. This force acting on the plunger launches the reservoir when the change of state of the first retention means occurs. This moves the micro needles against a patient's skin. The force of the means for storing energy acting on the plunger as well moves the plunger to expel the drug from the reservoir when the change of state of the second retention means occurs. It is hence obtained that one and the same energy storing means actuates both the insertion of micro needles into the skin, and the transdermal drug delivery. As a result, a more compact drug delivery device is obtained, which uses less material and is of a less complex structure, and which hence is more economical to produce compared to prior art devices.

Further advantageous aspects of the present invention are set out in the dependent claims.

Aspects of the invention will now be described in more detail with reference to the appended drawings, which are illustrative only and wherein same reference numbers indicate same features, wherein:
Figure 1 represents an axial cross sectional view of an assembly for drug delivery according to aspects of the invention in a ready-to-use position;
Figure 2 represents the device of Fig. 1 in a position wherein the launch member has been released from the housing and the plunger has been released from the launch member, such that the plunger has evacuated the drug from the reservoir;
Figure 3 represents an axial cross sectional view of yet another assembly according to aspects of the invention in a ready-to-use position;
Figure 4 represents the assembly of Fig. 3 in a position wherein the launch member has been released from the housing and the plunger has been released from the launch member, such that the plunger has evacuated the drug from the reservoir;
Figure 5 represents an exploded view of the assembly of Fig. 3 or Fig. 4;
Figures 6A-B represent axial cross sectional views taken at 90° from each other, of yet another assembly according to aspects of the invention in a ready-to-use position;
Figure 7 represents a cross sectional view of a Luer fitting between the patch with micro needles and the fluid reservoir, as can be used in assemblies of the invention;
Figure 8 represents a patch with micro needles with a needle for piercing a septum of the fluid reservoir, as can be used in assemblies of the invention;
Figure 9 represents a perspective view of a patch with micro needles;
Figures 10-13 represent perspective views of possible micro needles for use in assemblies of the invention, having different tip geometries.

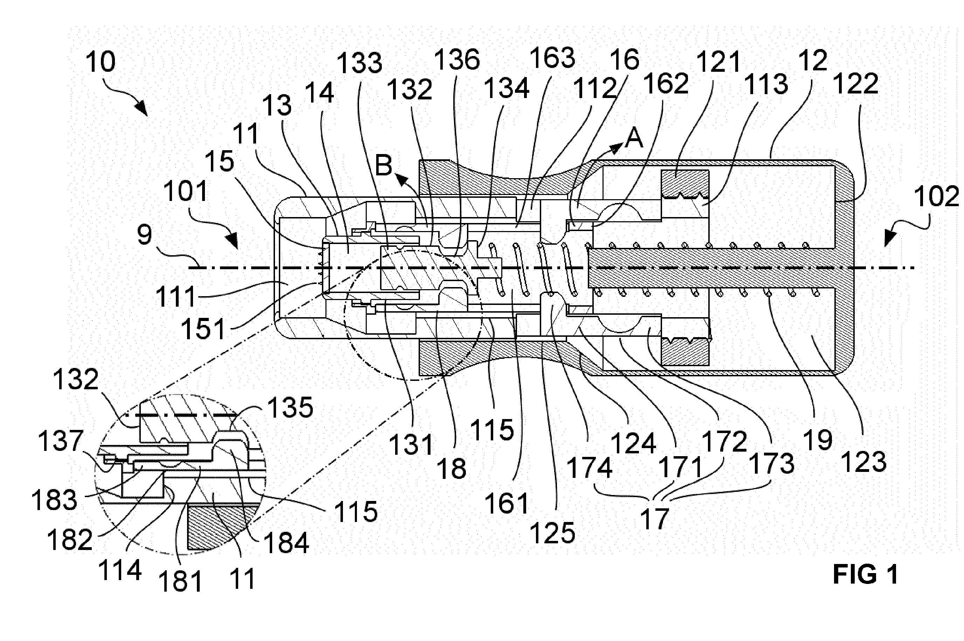

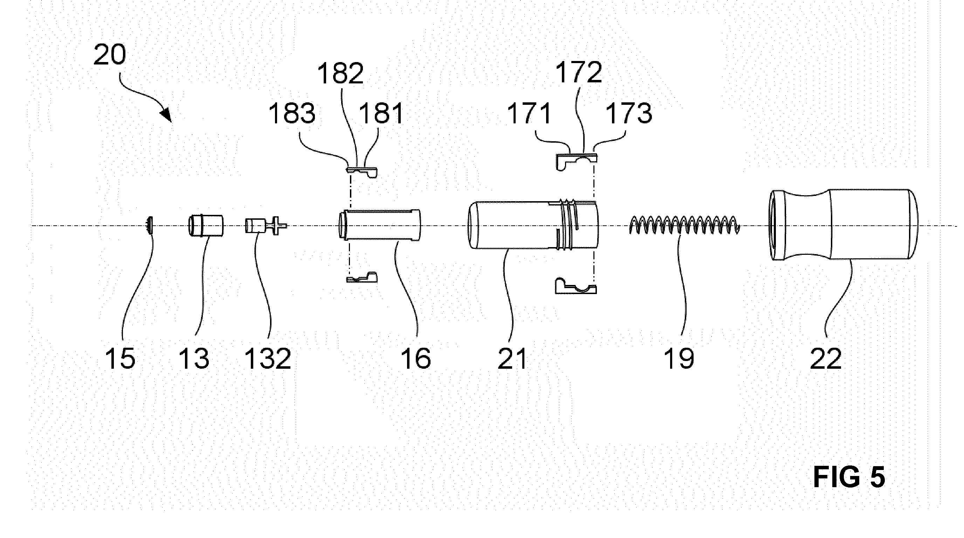

Referring to Figs. 1 and 5, a drug delivery device 10 according to aspects of the invention, also referred to as an assembly, comprises a housing 11, an actuation member 12, and a fluid reservoir 13 containing the drug or any other liquid formulation or composition to be administered transdermally. Housing 11 is advantageously of an essentially cylindrical shape, even though other shapes, such as prismatic with polygonal base (cross section), can be contemplated. Housing 11 is tubular, or hollow, with inner cavity 111 extending along an axis 9 of the device 10. Housing 11, and particularly inner cavity 111, is open to a distal end 101 of the device 10, which is the end arranged towards the skin of a person, and can be open to a proximal end 102 opposite the distal end. In the inner cavity 111, in proximity of the distal end 101, the housing 11 is arranged to receive the reservoir 13 in a manner as will be described.

The fluid reservoir 13 comprises a shell 131 enveloping the liquid formulation 14 which is to be administered. Shell 131 is advantageously of an elongated tubular shape, such as cylindrical, and advantageously thin walled. The shell 131 may, by way of example, be made of metal, glass, or a suitable polymeric or plastic material. The tubular/cylindrical shape of shell 131 advantageously extends with a longitudinal axis parallel to and possibly coincident with axis 9. Off-the-shelf liquid containers, such as a syringe or a capsule can be used as a reservoir 13. Alternatively, the reservoir 13 can be custom made.

A patch 15 provided with micro needles 151 is arranged at the distal end of the reservoir 13. The patch 15 can be formed as a cap which closes the tubular shell 131 at the distal end with the micro needles 151 providing fluid communication passages for the liquid formulation 14. It will be convenient to note that other arrangements of the coupling between patch 15 and reservoir 13 are possible and are described further.

A plunger 132 is arranged at a proximal end of the reservoir 13. Plunger 132 advantageously comprises a piston 133 at its distal end. Piston 133 is accommodated inside the tubular shell 131 and closes the reservoir 13 (i.e., the tubular shell 131) liquid tightly at the proximal end. Plunger 132 is advantageously arranged to move along axis 9, with piston 133 moving within the shell 131. When moving in distal direction relative to shell 131, piston 133 will exert pressure on the liquid formulation 14 such that the liquid formulation is expelled from the reservoir 13 through the micro needles 151.

Plunger 132 comprises a platform 134 at a proximal end thereof. A neck 136 connects the piston 133 with the platform 134. Neck 136 advantageously has a diameter which is smaller than a diameter of the piston 133 and smaller than a diameter of the platform 134, thereby forming a recess 135. Recess 135 can extend circumferentially along the plunger 132, about the axis of motion 9, though this is no requirement.

A launch member 16 is accommodated in the cavity 111 of the housing 11. Launch member 16 is advantageously tubular, with inner cavity (lumen) 161 and wall 162 advantageously extending along axis 9, and accommodates the fluid reservoir 13 and the plunger 132 inside cavity 161.

Advantageously, as shown in Fig. 1, the reservoir 13 is not completely received within the launch member 16, with patch 15 and possibly a portion of the reservoir 13 projecting distally of the launch member 16. Shell 131 is advantageously in an engaging relationship with the launch member 16 so as to prevent the reservoir shell 131 moving, at least in a proximal direction, relative to launch member 16, along axis 9. By way of example, reservoir 13 comprises a possibly circumferential ridge 137 projecting outwardly from the shell wall and fittingly engaging the launch member (e.g. wall 162). Alternatively, the shell can be threaded in the launch member. Other kinds of engagements for preventing movement of the reservoir shell, at least in a proximal direction, relative to the launch member are possible as known in the art.

According to an aspect of the invention, the device 10 comprises two retention means (or retention devices). A first retention means 17 holds the launch member 16 in position relative to the housing 11, along axis 9. A second retention means 18 holds the plunger 132, and hence the piston 133, in position relative to the launch member 16, along axis 9. Referring to Fig. 1, the first and second retention means comprise hinged tabs 171, 181 respectively. Hinged tabs 171 and 181 are advantageously arranged in pairs at opposite sides of the housing 11 and launch member 16 respectively. The hinges of the tabs 171, 181 can be formed by grooves or notches 172, 182 respectively, provided at base portions 173, 183 respectively, of the tabs. Alternatively, the tabs 171, 181 can be made sufficiently thin, such that they can swing on the base portions 173, 183. Tabs 171, 181 are accommodated in respective openings 112, 163 of the housing 11 and the launch member 16, and assembled/secured thereto at the base portions 173, 183.

Tabs 171, 182 comprise bosses 174, 184 respectively, at ends opposite the base portions 173, 183. Bosses 174 of first tabs 171 project in the inner cavity 111 of housing 11 to engage launch member 16 at opening 163 in wall 162. Bosses 184 of second tabs 181 project in the inner cavity 161 of launch member 16 to engage the plunger 132 in the recess 135.

Housing 11 can further comprise a fastening portion 113, arranged advantageously at a proximal end of housing 11. Fastening portion 113 retainingly engages with a corresponding fastening member 121 arranged within the actuation member 12. By way of example, the engagement between fastening portion 113 and member 121 can be a threaded engagement.

The actuation member 12 is advantageously formed as a handle, with a size allowing it to be easily held in a person's hand. The actuation member 12 advantageously comprises a distally open inner cavity 123, which receives the housing 11. Fastening member 121 is slidingly arranged within the inner cavity 123, so as to allow motion of member 121 relative to actuation member 12, along axis 9. A shoulder 124 projecting in cavity 123 defines a cavity wall portion 125 distal of shoulder 124 with reduced diameter and determines a distal position stop for fastening member 121. Shoulder 124 prevents that member 121 exits the cavity 123. A proximal position stop for member 121 is formed by the closed proximal end 122 of the cavity 123.

When assembled, fastening portion 113 is engaged in fastening member 121, and the housing 11 extends along cavity wall portion 125 and out of the handle 12 to the distal end 101. The cavity wall portion 125 has an inner diameter such that it prevents the tabs 171 to swing outwardly (as indicated by arrow A) on hinges 172. The axial position of cavity wall portion 125 advantageously corresponds to the axial position of the tabs 171 when the fastening member 121 is in a distal most position, e.g. at position stop against shoulder 124.

Likewise, the cavity 111 of housing 11 has a wall portion 115, with an inner diameter which prevents the second tabs 181 from swinging outwardly (as indicated by arrow B) on hinges 182. The axial position of wall portion 115 advantageously corresponds to the axial position of the second tabs 181 when the fastening member 121 is in a distal most position, e.g. at position stop against shoulder 124.

The height of shoulder 124 is however large enough to allow the swinging of tabs 171 along A once the fastening member 121 has moved (in relative terms) towards end 122 and the tabs 171 are located proximal of shoulder 124. A swinging along A releases the launch member 16 from the housing 11.

Another shoulder 114 is provided in the inner cavity 111 of housing 11 at a distal edge of the cavity wall portion 115. In the assembled position of Fig. 1, shoulder 114 is arranged distally of the second tabs 181. Shoulder 114 is arranged so as to increase the diameter of cavity 111 in the direction of the distal end 101. Shoulder 114 has a height which is large enough to allow the swinging of tabs 181 along B when the launch member 16 is released from the tabs 171, and hence from housing 11, and moves towards the distal end 101 and the tabs 181 move past shoulder 114. A swinging along B releases the plunger 132 from the launch member 16.

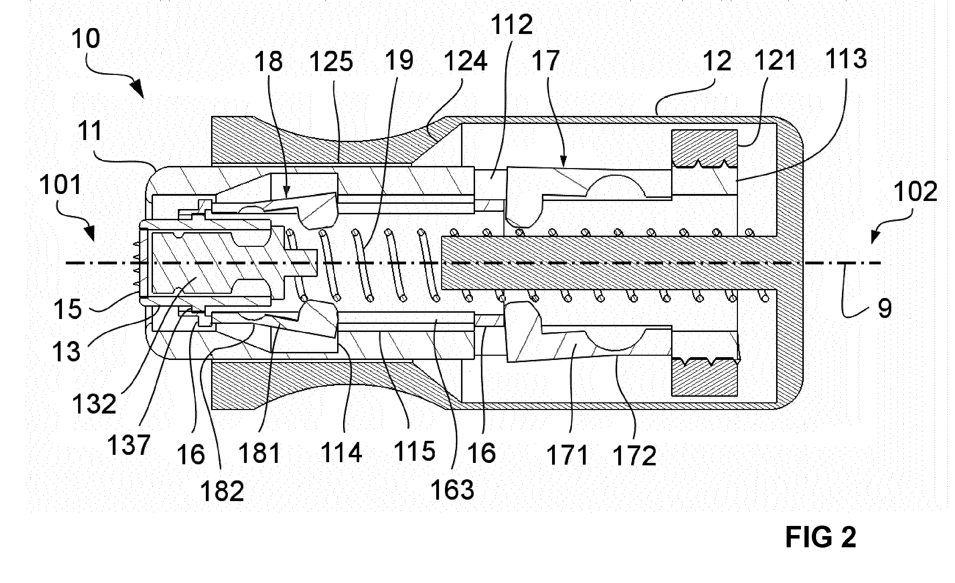

The swinging of the retention tabs 171, 181 along A and B respectively, from a straightened and retaining position (as shown in Fig. 1), to a swung open and releasing position (see Fig. 2) can be regarded as a change of state of these tabs. It will be convenient to note that a state in the present description does not need to be a stable state. By way of example, the swung open state of the tabs is labile, since typically the tabs will swing back to the straightened state as soon as the launch member or plunger respectively has moved along.

Since the launch member 16 cannot move relative to housing 11 while retained by the tabs 171, it will be appreciated that a swinging of tabs 181 along B, and hence a release of the plunger 132 must always occur after the swinging of tabs 171, and hence after a release of the launch member 16 from housing 11.

A spring 19, advantageously a coiled compression spring, engages the actuation member 12 on the one end, and the plunger 132 on the other end. Referring to Fig. 1, the coiled spring 19 is mounted within cavity 123, between the proximal end 122 and the platform 134. Depressing the actuation member 12 towards the distal end 101 (i.e., moving it down relative to housing 11, towards the skin) compresses (loads) the spring 19, such that energy is stored in it. Spring 19 can be suitably pre-loaded in the assembled device in order to bias the member 121 against shoulder 124, which ensures that the parts are held in position and prevents undesired swinging of retaining tabs 171 or 181. Such a pre-loading of spring 19 can be obtained by choosing an appropriate length for the coiled compression spring 19.

According to an aspect of the invention, the loaded spring acts on the plunger 132 and provides the driving force to expel the liquid 14 out of reservoir 13, as well as the driving force for launching the member 16 and hence for launching the patch 15 of micro needles against the skin. It is hence obtained that one and the same energy storing device, such as the spring 19, can advantageously actuate both the insertion of micro needles, and the liquid delivery.

A suitable selection of the spring 19 and control of the release time/position of launch member 16 allows for obtaining a controlled impact velocity of the patch 15 against the skin and/or a controlled impact force. The impact velocity is advantageously between 0.1 m/s and 10 m/s. After skin penetration, the spring 19 will also maintain the patch correctly against the skin during drug delivery, which further avoids leakage.

Additionally, spring 19 can provide a driving force for a swinging motion of tabs 171 along A and tabs 181 along B. This can easily be obtained by appropriate shaping of the bosses 174 and 184, such as making them curved or tapered at the engagement faces, as known in the art. Alternatively, it is possible to form tabs 171 and 181 as leaf springs and to bias them to perform the swinging motion A, B respectively upon passing shoulders 124, 114 respectively.

It will be convenient to note that the spring 19 can be replaced by any other means able to store energy and to release the stored energy as a driving force on the plunger 132 when desired, such as other elastic elements, e.g. a leaf spring, or hydraulic or pneumatic cylinder/plunger arrangements. Furthermore, it is not required that the energy storing means (the spring 19 in Fig. 1) be operably coupled to the actuation member 12, since the energy storing means 19 can be provided in a pre-loaded state in the drug delivery device 10 with no need of further loading it by moving (depressing) the actuation member 12. In such case, a suitable alternative is to secure the energy storing means 19 to the housing 11 and operably couple it to plunger 132 in a pre-loaded state. By so doing, as soon as retention tabs 171 release the launch member 16, the energy storing means 19 will release energy on the plunger to launch the member 16 and hence the patch 15 of micro needles against the skin. Such an arrangement would be particularly suitable when hydraulic or compressed gas (pneumatic) cylinders are used as energy storing means, since they can be loaded beforehand. In such a case, the motion of the actuation member 12 serves to trigger the change of state, such as the swinging, of the retention tabs 171, 181, or to activate a latch for triggering the change of state of the retention tabs once the actuation member assumes a depressed position relative to the housing.

Referring now to Fig. 2, the launching motion of member 16 - once released from tabs 171 - also moves tabs 181 past shoulder 114. Either through the spring force, or through the bias of tabs 181, or both, as the case may be, tabs 181 swing open as shown in Fig. 2, and release the plunger 132. The spring 19 now acts on the plunger 132 to move the piston 133 down the reservoir 13 and expel the liquid formulation 14 through the micro needles 151.

According to aspects of the invention, hence, one and the same spring 19 provides for a controlled force required to make the micro needles impact against the skin, and for the force required to move the plunger to empty the reservoir and administer the liquid formulation through the micro needles. This makes devices according to the invention much simpler in construction and hence more economical. This aspect is particularly important when the device is partly or completely disposable.

The spring force available for launching member 16, and hence for impacting the patch of micro needles against the skin, can be controlled by defining an appropriate initial pre-load for spring 19, or by appropriately selecting the instant of time of swinging tabs 171 and releasing launch member 16. By way of example, the instant of time can be adjusted by appropriate selection of the position of shoulder 124 along axis 9. Alternatively, instead of making first tabs 171 swing automatically once past shoulder 124, it is possible to couple tabs 171 to a latch (not shown) which can be provided on the actuation member 12, which operates manually or automatically. This makes it possible to first completely depress actuation member 12 on housing 11, and then trigger the latch - either manually, or automatically - to swing tabs 171 and release launch member 16.

All moveable parts, viz. the actuation member 12, the launch member 16, and the piston 132 are advantageously arranged and configured for moving concentrically, on axis 9, even though this is not an absolute requirement.

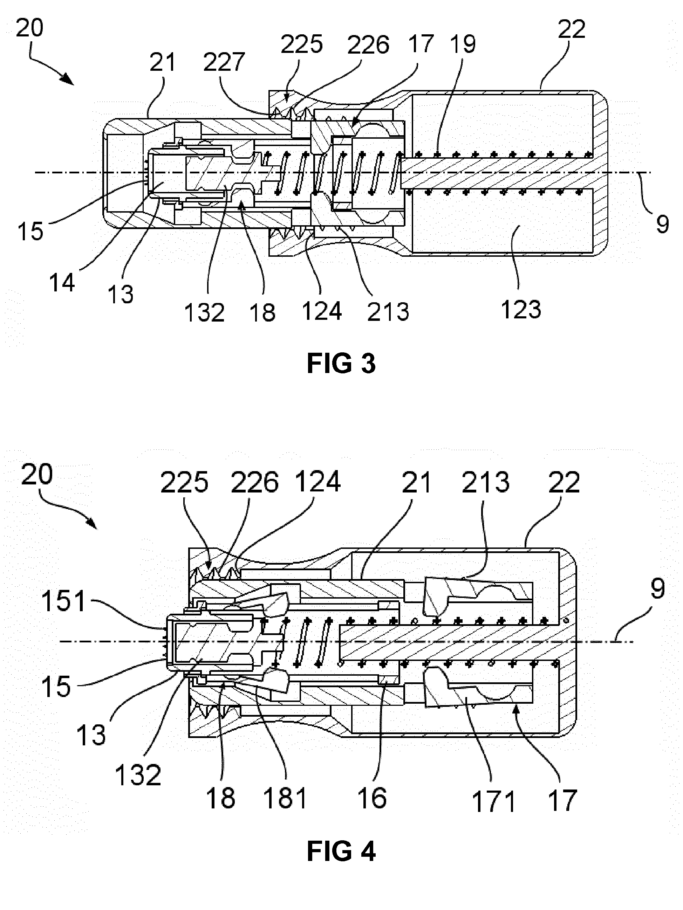

Figs. 3 and 4 show a drug delivery device 20 which differs from device 10 only in the fastening arrangement between housing 21 and actuation member 22. Device 20 does not comprise the fastening member 121 as in device 10. Instead, the cavity wall portion 225 distal of shoulder 124 is provided with grooves 226 and ridges 227. The housing 21 comprises projecting ridges 213 which are configured for engagement with ridges 227 of the actuation member 22. The ridges 227 and grooves 226 on the one hand, and the ridges 213 on the other hand can extend helically about axis 9, as shown in Fig. 3. Alternatively, they can be circular. The size of the ridges 213 of the housing 21 is such that they snugly fit between the ridges 227 of the actuation member 22. The engagement between ridges 227 and 213 ensure that the housing 21 is retained in the actuation member 22 once slid (or turned as the case may be) into the cavity 123 of actuation member 22. In addition, the engagement between ridges 227 and 213 is sufficiently weak, such that it allows to depress the actuation member 22 relative to the housing 21, along axis 9. Ridges 213 can be thought of as small laminae withholding the housing 21 inside the cavity 123 of the actuation member 22.

To use the device 10 or 20, and referring to Figs. 1 and 5, launch member 16 can be pre-assembled by inserting reservoir 13 with plunger 132 in the cavity 161, until tabs 181 engage recess 135 of plunger 132. Housing 11 can be pre-assembled by inserting pre-assembled launch member 16 inside cavity 111 of housing 11 until tabs 181 engage openings 163 of launch member 16. Pre-assembly of launch member 16 and/or pre-assembly of housing 11 can be performed at a factory site, or by a person who administers the liquid formulation 14. Next, the housing 11 is fastened in the actuation member 12, which already contains spring 19. By way of example, housing 11 can be fastened by threading portion 113 in fastening member 121 provided in actuation member 12. Fastening of housing 11 to actuation member 12 may or may not pre-load the spring 19.

The device 10 is now ready for use. A patient or assistant holds the actuation member 12 and brings the distal end 101 of the housing 11 against the skin of the patient. At the distal end 101, the housing can be suitably provided with a rubber sleeve or other device for tensioning the skin, as known in the art. Depressing actuation member 12 towards the skin (towards end 101) further loads the spring 19. It also moves shoulder 124 past tabs 171. Either through the spring force, or through the bias of tabs 171, or both, as the case may be, tabs 171 swing open as indicated by arrow A, and release the launch member 16. At this moment, the tabs 181 are still in position retaining plunger 132. By consequence, the force of compressed spring 19 now acts on the launch member 16 as a whole, through plunger 132 and tabs 181, hence launching member 16 against the skin. It will be convenient to note, that in the assembled position, micro needles 151 advantageously project from launch member 16, so that the release of member 16 causes the micro needles 151 to impact the skin and penetrate it. The frictional engagement of a ridge 137 with launch member 16 prevents that the reservoir 13 (shell 131) would move towards the piston 133.

It will be convenient to note that the hinges 172, 182 can be replaced by pivotal axes attached to the housing or the launch member, respectively. The retention tabs 171, 181 would hinge on the pivotal axes in such case, which would additionally provide for securing the tabs to the housing or launch member. Hence, the base members 173 and 183 would not be necessary in this case.

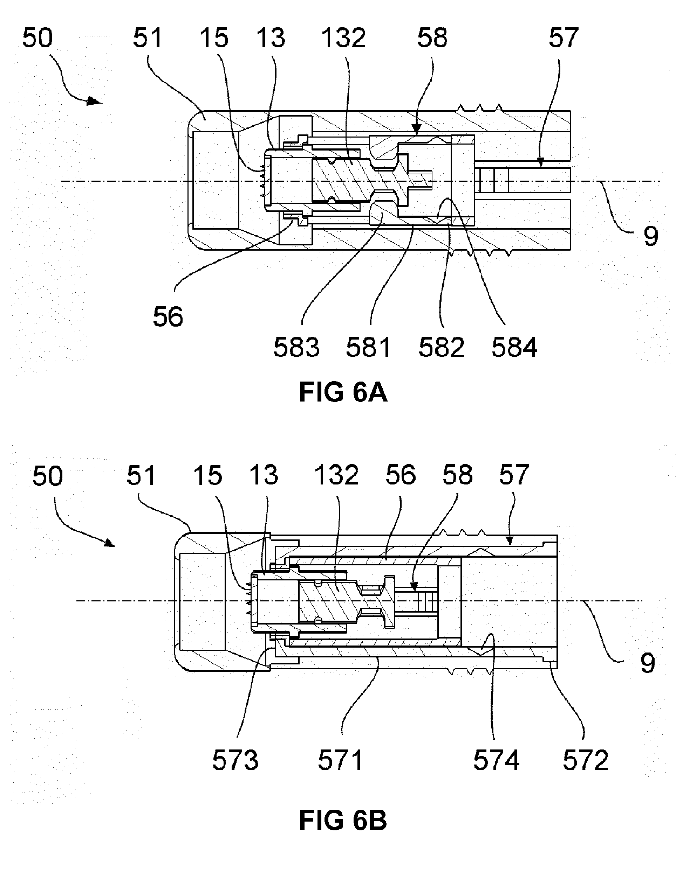

In devices 10 and 20, the swinging of retention means 17 and 18, and hence the release of the launch member 16, and the plunger 132, is controlled by a constructional arrangement of the shoulders 124 and 114. Alternatively, it is possible to control the release of launch member 16 and/or plunger 132 only based on the amount of loading (i.e., compression in case of a compression spring) of the spring 19. This can be obtained by replacing either one or both hinged tabs 171 and 181 with frangible tags as shown in Figs. 6 A-B.

Referring to Figs. 6A-B, drug delivery device 50 differs from device 20 only in the construction of the first and second retention means. For ease of representation, the actuation member 22 is not represented in Figs. 6A-B. The first retention means 57 holds the launch member 56 in position relative to housing 51. The second retention means 58 holds the plunger 132 in position relative to launch member 56. In devices 10 and 20, the first and second retention means are coplanar (they are arranged in a same cross sectional plane). It will be convenient to note that this need not be the case and they can be arranged at angled axial planes. By way of example, in device 50 the first and second retention means are arranged in two perpendicular axial planes (the cross sections of Fig. 6A and Fig. 6B are at 90°). First and second retention means 57, 58 comprise pairs of frangible tags 571, 581 respectively.

First tags 571, shown in cross section in Fig. 6B, are secured to the housing 51 through a proximal attachment portion 572 and are secured to the launch member 56 through a distal attachment portion 573. The proximal attachment portion 572 is arranged proximally of the distal attachment portion 573, as considered along axis 9. First tags 571 further comprise a point of weakness 574 interposed between the proximal attachment portion 572 and the distal attachment portion 573. The point of weakness 574 is a frangible portion of tag 571, such as a notch, or other suitable construction as known in the art.

Second tags 581, shown in cross section in Fig. 6A, are secured to the launch member 56 through a proximal attachment portion 582 and retainingly engage the plunger 132 through a distal attachment portion 583. The proximal attachment portion 582 is arranged proximally of the distal attachment portion 583, as considered along axis 9. As with first tags, second tags 581 further comprise a point of weakness 584 interposed between the proximal attachment portion 582 and the distal attachment portion 583.

The point of weakness 584 is a frangible portion which is stronger than the point of weakness 574 of tag 571, i.e. it is configured to break at a higher tensile load. That is, when actuation member 12 is depressed, spring 19 is compressed and exerts a force on plunger 132, which is transmitted to launch member 56 through the second tag 581 and from launch member 56 to the housing 51 through first tag 571. As a result, both the first and second tags are subjected to a tensile load which causes a stress concentration in the frangible portions 574 and 584. When frangible portion 574 is made weaker than frangible portion 584, the first tag 571 is configured to break (at point 574) prior to rupture of the second tag 581 (at point 584). This has a same effect as with tabs 171 and 181, i.e. the launch member 56 will be released before releasing plunger 132 from launch member 56.

It will be convenient to note that tabs 171, 181 and tags 571, 581 can be combined as desired.

Even though launch members 16, 56 have been described as separate from the fluid reservoir 13, this need not be so, and aspects of the invention contemplate cases in which the launch member and the fluid reservoir form an integral unit, that is, the shell 131 and the launch member 16, 56 are integral, or unitary.

In the previous figures, the patch 15 of micro needles 151 has been represented as an end cap in permanent fluid communication with the reservoir 13. It will be convenient to note that this need not be so, and it is possible to provide a check valve (not shown) between the reservoir 13 and the patch 15 with a suitable cracking pressure specification (i.e., the pressure level at which the valve opens).

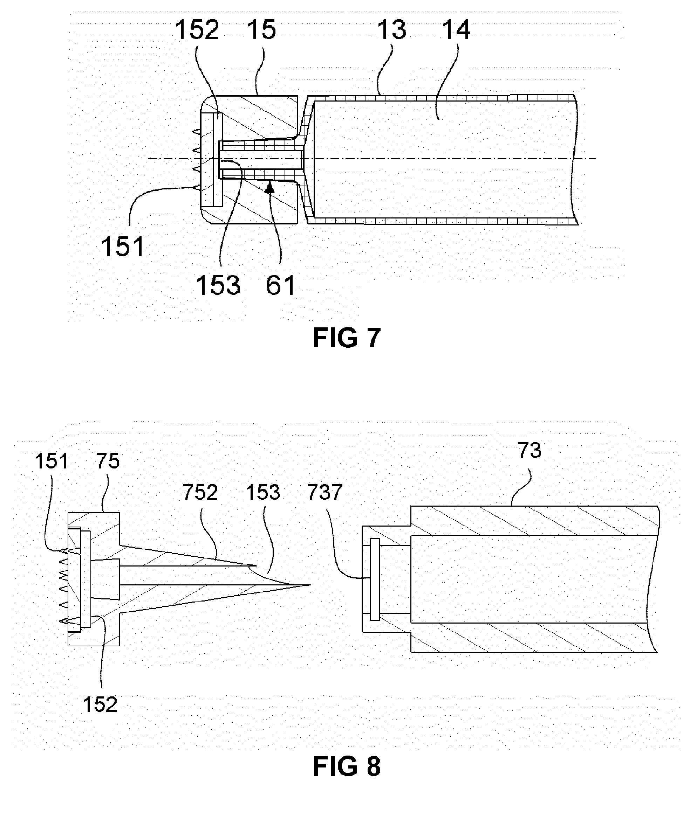

Patch 15 can be glued or welded at the end of reservoir 13. Alternatively, the fluid connection between reservoir and patch can advantageously be made through a Luer taper fitting 61, such as a Luer Lock (or Luer Lok®), as shown in Fig. 7. These kinds of fittings provide a leak-free connection and are economical. Alternatively, the patch of micro needles and the reservoir 13 are only arranged to enter in fluid communication upon loading spring 19. This can be obtained as shown in Fig. 8 by providing the patch 75 with micro needles 151 at a distal end, and with a projecting needle 752 at a proximal end. The reservoir 73 is distally closed by septum 737 which is pierceable by needle 752. The device is assembled such that the needle 752 is oriented facing septum 737. Patch 75 may be secured to launch member 16 and reservoir 73 may be slidable, or vice versa. Upon loading spring 19, the force exerted on the plunger 132 will move reservoir 73 against needle 752 to pierce septum 737 and create a fluid communication between micro needles 151 and reservoir 73.

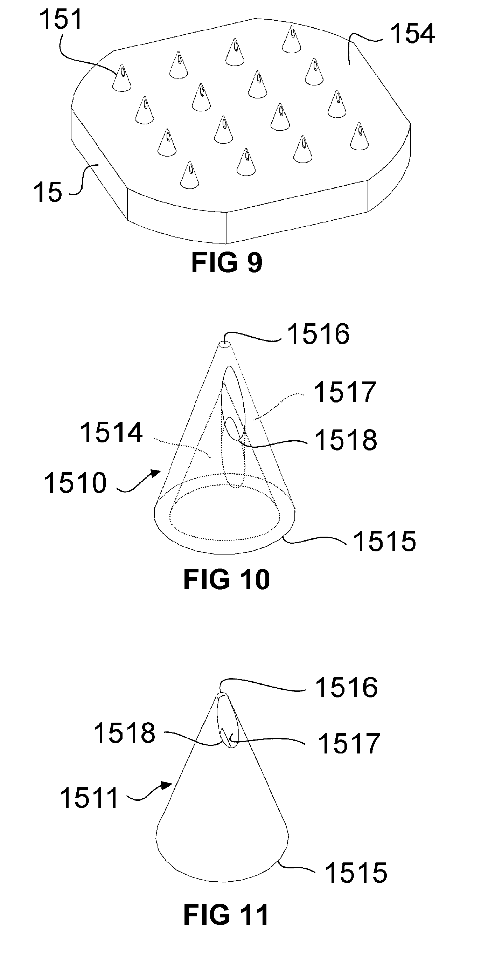

Referring to Figs. 7 and 8, the patch is advantageously formed as a body 15, 75 enclosing a distribution manifold 152 in fluid communication with the micro needles 151. Manifold 152 serves to distribute the liquid formulation or drug across the micro needles 151. The patch advantageously comprises an inlet port 153 for the liquid formulation 14 and in fluid communication with the manifold 152. A perspective view of the front side of a possible patch 15 with projecting micro needles 151 is shown in Fig. 9. This patch comprises a regular array of four by four micro needles 151.

Micro needles 151 are advantageously hollow with an inner lumen having a diameter suitably between 10 µm and 200 µm. Micro needles 151 can have a height (projection as measured from a base of the patch) advantageously between 100 µm and 1.5 mm, advantageously smaller than 1 mm. The height of the micro needles is selected such that they at least enable to penetrate or pierce the stratum corneum of the skin. They can be made of a polymeric or plastic material, such as polycarbonate, or made of metal or silicon. The patch 15, 75 can comprise an array of up to 100 micro needles.

Suitable micro needle geometries are represented in Figs. 10 to 13. Micro needles 1510-1513 advantageously have a conical (internal) cavity 1514 extending from a base 1515 of the micro needle towards a tip 1516 of the micro needle. The micro needle base 1515 corresponds to the level of the outer surface 154 of patch 15. The conical cavity 1514 is enclosed by a shell or wall 1517 of the micro needles, which advantageously has a conical external surface, extending between base 1515 and tip 1516. The external surface of the micro needle is advantageously of right circular conical shape. An orifice 1518 extends through the wall 1517 providing an outlet for cavity 1514 from which the drug is ejected / injected in the patient's skin. The conical cavity 1517 ensures correct homogeneous Hagen-Poiseuille flow of the drug along the micro needle. The orifice 1518 advantageously has a diameter between 10 µm and 200 µm. The tip 1516, which can be flat and result from e.g. truncation of the external conical surface at the top due to production constraints, advantageously has a surface area of 3000 µm² or less, advantageously 2500 µm² or less. The micro needle's external surface hence can resemble a truncated cone.

Micro needles used in assemblies according to the invention can be injection moulded or fabricated by two-photon polymerization. Micro needles so obtained can be blind at first, i.e. without orifice 1518. The orifice 1518 can be formed in a subsequent manufacturing step, such as through removing material from wall 1517 by laser. A mask can be used, allowing several orifices, such as the orifices of all the micro needles 151 on a same patch 15, to be manufactured in one set of laser shots. The laser can be an excimer laser.

Additional details on suitable shapes of the micro needles and on possible processes of manufacture are described in WO 2012/126784, in particular Figs. 8-11 and corresponding description.

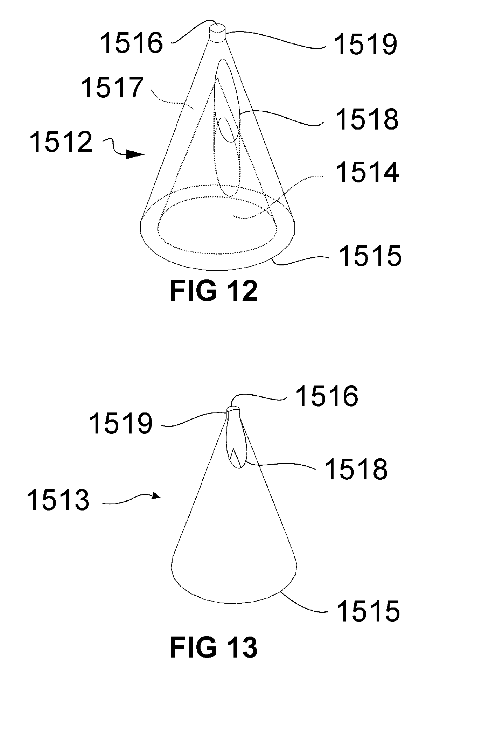

A laser can be used to sharpen the micro needle tip, either in a separated operation, or at the same time as creating the orifice. Sharpening removes material adjacent the tip 1516 and reduces the tip surface area: compare micro needles between Figs. 10 and 11 and between Figs. 12 and 13, where micro needles 1511 and 1513 of respectively Fig. 11 and 13 have sharpened tips.

The tip can comprise a cylindrical projection 1519 on top of the truncated cone shape, as shown in Fig. 12. Such a projection facilitates moulding and demoulding operation. As long as the tip surface area is sufficiently small, skin penetration is not hampered and production cost can be kept low.

The amount of liquid formulation to be injected should be sufficient to allow an injection time of 1 minute or less. Suitable volumes that can be injected in such time frames may be comprised between 0.1 ml and 3 ml. Needless to say, the reservoir 13 will suitably have a corresponding content (volume). It will be convenient to note that the drug delivery devices according to the invention need to be maintained on the skin, with the actuation member 12, 22 depressed during the entire injection time. As spring 19 is acting both to press the patch 15 against the skin, and to move the piston 133 down the reservoir 13, it will be convenient to note that devices of the invention can advantageously be used in any orientation.

Most parts of the drug delivery devices described herein, except for the reservoir 13, and the spring 19, can be made of common polymeric or plastic materials, such as but not limited to injection mouldable materials.

Drug delivery devices can be used with any suitable (liquid) drug or formulation or composition that can be administered by hypodermic injection. This can be any substance, pharmaceutical, nutriceutical, cosmeceutical, or therapeutic agent. Examples of drugs may be selected from one or more of the following non limiting list: Omalizumab, Alemtuzumab, Sevcizumab, (Medi-507, CaCP29-humMaB), Cankinumab, (GSK2800528), (CNT0888), (ALD403), Ofatumumab, Cixutumumab, (Medi-522), Sirukumab, Siltuximab, Bortezomib, Ranibizumab, (anti CD45 monoclonal antibody), (Medi-1814), Pagibaximab, Adalimumab, Simtuzumab, Golimumab, Sonepcizumab, Ipilimumab, (anti IL-12 monoclonal antibody), Rituximab, Epratuzumab, (BMS-981164), Tigatuzumab, Infliximab, Tralokimumab, (Medi-493), Ustekinumab, Alirocumab, (GSK933776), Bevacizumab, Cetuximab, Pazopanib, Palivizumab, Doxorubicin hydrochloride, Innotecan hydrochloride, Etoposide, Cyclophosphamide, Dactinomycin, Temozolomide, Vincristine sulfate, Ifosfamide, Melphalan, Prednisone Dexrazoxan hydrochloride Methotrexate, Abraxane.

## Claims

1. Assembly (10, 20, 50) for drug delivery, comprising:
a plurality of micro needles (151),
a reservoir (13) containing the drug (14) and arranged to be fluidly coupled to the micro needles,
a plunger (132) coupled to the reservoir,
a housing (11, 21, 51) arranged to receive the reservoir,
means (19) for releasably storing energy,
first retention means (17, 57) for securing the reservoir to the housing and configured to change state to release the reservoir, and second retention means (18, 58) for securing the plunger to the reservoir and configured to change state to release the plunger,
**characterised in that** the means (19) for storing energy are operably coupled to the plunger (132) to provide a force on the plunger for launching the reservoir (13) when the first retention means change state and for ejecting the drug when the second retention means change state.

2. Assembly of claim 1, wherein the reservoir (13) is arranged to move relative to the housing (11, 21, 51) and the plunger (132) is arranged to move relative to the reservoir along coincident axes (9).

3. Assembly of claim 1 or 2, wherein the second retention means (18, 58) are configured to change state following the change of state of the first retention means (17, 58).

4. Assembly of any one of the preceding claims, comprising an actuation member (12, 22) moveable relative to the housing along a predetermined path (9), wherein the means (19) for storing energy is operably coupled to the actuation member (12, 22), such that the actuation member is configured to load the means (19) for storing energy when the actuation member is moved along the predetermined path.

5. Assembly of claim 4, wherein the predetermined path (9) of the actuation member is axially aligned with a direction of motion of the plunger (132).

6. Assembly of claim 4 or 5, wherein the actuation member (12, 22) is configured for triggering the change of state of the first retention means (17) to release the reservoir (13) when the actuation member is moved along the predetermined path (9).

7. Assembly of any one of the claims 4 to 6, wherein the actuation member (12, 22) is arranged at a proximal end (102) of the device and the micro needles (151) are arranged at a distal end (101) of the device, configured to face a patient's skin, and wherein the first retention means (17) are arranged proximal of the second retention means (18).

8. Assembly of any one of the claims 4 to 7, wherein the actuation member (12, 22) comprises an internal cavity (123), and wherein the housing (11, 21, 51) is slidably engaged in the internal cavity (123).

9. Assembly of any one of the preceding claims, wherein the first and/or the second retention means (17, 57, 18, 58) comprise a first member (173, 572, 183, 582) attached to respectively the housing (11, 21, 51) and the reservoir (13), and a second member (171, 571, 181, 581) attached to respectively the reservoir (13) and the plunger (132), wherein the first and second members are coupled to each other through a hinge (172, 182) or a point of weakness (574, 584).

10. Assembly of any one of the claims 4 to 8, wherein the first retention means (17) comprise first hinged retention tabs (171) coupling the reservoir (13) to the housing (11, 21) and configured to release the reservoir from the housing by a swinging motion (A) on a hinge (172) of the first retention tabs, and wherein the actuation member (12, 22) comprises a surface portion (125) adjacent the retention tabs preventing the swinging motion, and a shoulder (124) adjacent the surface portion (125) providing space for allowing the swinging motion when the actuation member (12, 22) is moved such that the shoulder (124) moves past the retention tabs.

11. Assembly of any one of the preceding claims, wherein the second retention means (18) comprise second hinged retention tabs (181) coupling the plunger (132) to the reservoir (13) and configured to release the plunger from the reservoir by a swinging motion (B) on a hinge (182) of the second retention tabs, and wherein the housing (11, 21) comprises a second surface portion (115) adjacent the second retention tabs preventing the swinging motion, and a shoulder (114) adjacent the second surface portion providing space for allowing the swinging motion when the reservoir (13) moves past the shoulder upon the reservoir being released from the housing.

12. Assembly of claim 10 or 11, wherein the hinge (172, 182) of the first or second retention tabs (171, 181) is obtained by a notch or groove in the retention tab.

13. Assembly of any one of the claims 10 to 12, wherein the means (19) for storing energy is configured to drive the swinging motion of the first or second retention tabs (171, 182) on their respective hinge (172, 182) through release of energy on the plunger (132).

14. Assembly of any one of the preceding claims, wherein the means (19) for storing energy is a spring, such as a coiled compression spring.

15. Assembly of any one of the preceding claims, comprising a launch member (16, 56) coupled to the reservoir (13) and receivable in the housing (11, 21, 51), wherein the first retention means (17, 57) are arranged to releasably attach the housing to the launch member, and wherein the second retention means are arranged to releasably attach the launch member (16, 56) to the plunger (132).
